# EUROPÄISCHE PATENTANMELDUNG

(11) **EP 1 676 568 A1**
(43) Veröffentlichungstag der Anmeldung: **05.07.2006**
(21) Anmeldenummer: 04031036.9
(22) Anmeldetag: 30.12.2004
(51) Int. Cl.: A61K 9/127

(54) **Phospholipide enthaltende Teilchen sowie deren Herstellung und Verwendung als Arzneimittel**

(71) Anmelder: MERCKLE GMBH, D-89079 Ulm (DE)
(72) Erfinder: Schneider, Peter Dr., 89077 Ulm (DE); Rose, Peter Dr., 88441 Mittelbiberach (DE); Ibscher, Bernd Dr., 89143 Blaubeuren (DE); Schock, Irene Dr., 89134 Blaustein (DE)
(74) Vertreter: Breuer, Markus

(57) **Zusammenfassung**

Die Erfindung betrifft eine Zusammensetzung, enthaltend eine wässrige Lösung (i) und ein Phospholipidgemisch (ii), enthaltend Phosphatidylcholin (ii-1), Phosphatidylethanolamin (ii-2) und gegebenenfalls Sphingomyelin (ii-3), wobei das Phospholipidgemisch in Form von Teilchen, bevorzugt in Form von Liposomen, mit einem mittleren Teilchendurchmesser von 15 bis 250 Nanometer (nachfolgend als nm bezeichnet) in der wässrigen Lösung vorliegt, sowie ein Verfahren zur Herstellung der erfindungsgemäßen Zusammensetzung und die Verwendung der erfindungsgemäßen Zusammensetzung als Arzneimittel, insbesondere als Arzneimittel zur Behandlung von Osteoarthrose.

## Beschreibung

Die Erfindung betrifft eine Zusammensetzung, enthaltend eine wässrige Lösung (i) und ein Phospholipidgemisch (ii), enthaltend Phosphatidylcholin (ii-1), Phosphatidylethanolamin (ii-2) und gegebenenfalls Sphingomyelin (ii-3), wobei das Phospholipidgemisch in Form von Teilchen, bevorzugt in Form von Liposomen, mit einem mittleren Teilchendurchmesser von 15 bis 250 Nanometer (nachfolgend als nm bezeichnet) in der wässrigen Lösung vorliegt, sowie ein Verfahren zur Herstellung der erfindungsgemäßen Zusammensetzung und die Verwendung der erfindungsgemäßen Zusammensetzung als Arzneimittel, insbesondere als Arzneimittel zur Behandlung von Osteoarthrose.

Die Verwendung von Phospholipiden als oberflächenaktive Mittel ist bekannt. Aus WO 89/1777 sind beispielsweise Gelenkflüssigkeiten für Osteoarthritis und Rheumaerkrankungen bekannt, welche die Phospholipide Phosphatidylcholin (PC), Phosphatidylethanolamin (PE) und Sphingomyelin (Sph) und Hyaluronsäure in einer Kochsalzlösung enthalten. Der Einsatz von Hyaluronsäure ist jedoch nicht unbedenklich, beispielsweise da Hyaluronsäurepräparate verunreinigt sind und Hyaluronsäure daher häufig unerwünschtes immunogenes/inflammatorisches Potential besitzt.

WO 91/12026 offenbart deshalb ein Mittel zur Behandlung postoperativer Adhäsion, welches die Phospholipide DPPC, PE und Sph (ohne Mengenangaben) in Propylenglykol gelöst enthält. Ferner sind aus WO 97/22345 Gelenkflüssigkeiten für Osteoarthritis bekannt, welche die Phospholipide DPPC, PE und Sph (ohne Mengenangaben) und Propylenglykol enthalten. Der Einsatz von Propylenglykol gilt jedoch bei zahlreichen medizinischen Anwendungen als unerwünscht, da es Irritationen und lokale Reizungen im Gelenk auslösen kann.

Aus WO 91/12808 sind außerdem künstliche Tränenflüssigkeiten bekannt, welche die Phospholipide DPPC, PE und Sph (ohne Mengenangaben) in Propylenglykol oder Kochsalz als Trägerflüssigkeit enthalten.

Aufgabe der Erfindung war es daher, eine Zusammensetzung bereit zu stellen, die ohne Zusatzstoffe wie Hyaluronsäure oder Propylenglykol vorteilhafte Eigenschaften, bevorzugt vorteilhafte Eigenschaften als Arzneimittel, insbesondere zur Behandlung von Osteoathrose, besitzt.

Insbesondere war es Aufgabe der Erfindung, eine Zusammensetzung bereit zu stellen, die einerseits die Reibung zweier Flächen reduziert, und zwar vorteilhafter als Hyaluronsäure, die anderseits nur körpereigene Substanzen oder solche Substanzen, die üblicherweise mit der Nahrung aufgenommen werden, enthält, und die zudem lagerstabil ist, bevorzugt lagerstabil bei Raumtemperatur. Ferner sollte sich die Zusammensetzung bei entsprechender medizinischer Verwendung gut an Knorpel anlagern können.

Bevorzugt war es ferner die Aufgabe, eine Zusammensetzung bereit zu stellen, die bei Körpertemperatur in flüssigkristalliner Form vorliegt,
ein schlechtes Substrat für PLA₂ darstellt,
durch welche eine lange Verweildauer im Gelenk gegeben ist, und/oder
die kein immunogenes oder inflammatorisches Potential besitzt und keine Irritationen oder lokalen Reizungen im Gelenk auslöst.

Gegenstand der Erfindung ist daher eine Zusammensetzung, enthaltend
(i) eine wässrige Lösung und
(ii) ein Phospholipidgemisch, enthaltend, bezogen auf das Gesamtgewicht des Phospholipidgemisches (ii),
(ii-1) 60 bis 95 Gewichtsprozent (Gew.-%) Phosphatidylcholin,
(ii-2) 2 bis 25 Gew.-% Phosphatidylethanolamin und
(iii-3) 0 bis 15 Gew.-% Sphingomyelin,
wobei das Phospholipidgemisch in Form von Teilchen mit einem mittleren Teilchendurchmesser von 15 bis 250 nm, bevorzugt 30 bis 150 nm, insbesondere 50 bis 130 nm, in der wässrigen Lösung vorliegt.

In einer bevorzugten Ausführungsform handelt es sich bei den Teilchen mit einem mittleren Teilchendurchmesser von 15 bis 250 nm um Liposome.

Grundlage für die verwendete wässrige Lösung (i) bildet Wasser, bevorzugt Wasser für Injektionszwecke. Bei Wasser für Injektionszwecke handelt es sich üblicherweise um Wasser, das gemäß Monographie Ph. Eur. 1997 hergestellt wurde.

In einer bevorzugten Ausführungsform liegt die wässrige Lösung (i) in einem pH-Bereich von 6 bis 9, besonders bevorzugt in einem pH-Bereich von 6,5 bis 8, insbesondere von 6,8 bis 7,6.

Die wässrige Lösung (i) kann nur aus Wasser bestehen. Alternativ kann die wässrige Lösung (i) Salze enthalten. Geeignete Salze können beispielsweise Natrium, Kalium, Magnesium und/oder Calcium als Kationen und Chlorid, Sulfat, Phosphat, Hydrogenphosphat und/oder Dihydrogenphosphat enthalten.

Ferner ist die wässrige Lösung (i) bevorzugt in einem pH-Bereich von 6 bis 9 gepuffert. Unter "gepuffert" ist hierbei zu verstehen, dass sich der pH-Wert der Lösung bei Zugabe von Wasserstoff- oder Hydroxidionen zunächst nur geringfügig ändert, da die zugegebenen Wasserstoff- oder Hydroxidionen, beispielsweise durch Bildung schwacher Säuren und Basen, abgefangen werden.

Zur Herstellung der gepufferten wässrigen Lösung (i) eignen sich im allgemeinen Salze aus schwachen Säuren und starken Basen oder Salze von starken Säuren und schwachen Basen. Bevorzugt können Puffer auf Basis von Phosphaten, Hydrogenphosphaten und/oder Dihydrogenphosphaten und Phosphorsäure verwendet werden. Besonders bevorzugt wird ein Puffer auf Basis von Alkalidihydrogenphosphat und Phosphorsäure verwendet. Insbesondere wird ein Puffer, enthaltend Kaliumdihydrogenphosphat und Phosphorsäure verwendet.

In einer weiteren bevorzugten Ausführungsform wird ein Puffer, umfassend Phosphorsäure und Tris(hydroxymethyl)aminomethan (im Fachgebiet bekannt als "Tris", CAS 77-86-1) verwendet.

Im Rahmen der vorliegenden Erfindung kann es zur Stabilisierung der Liposomen vorteilhaft sein, dass die wässrigen Lösung (i) möglichst wenig "kleine" Kationen der Alkali- und keine Kationen der Erdalkaligruppe enthält. In einer bevorzugten Ausführungsform enthält die wässrige Lösung (i) Lithium-, Natriumionen in einer Menge von weniger als 1000 ppm, mehr bevorzugt von weniger als 100 ppm, insbesondere weniger als 10 ppm.

Das Phospholipidgemisch (ii) enthält Phospholipide, die im Fachgebiet auch unter den Begriffen "Phosphoglyceride" oder "Phosphatide" bekannt sind. Zu dieser Klasse von Naturstoffen rechnet man fettähnliche Triglyceride, die zwei, bevorzugt langkettige, Fettsäuren und einen Phosphorsäurerest, an den eine Base gebunden ist, enthalten.

Phospholipide kommen in zahlreichen tierischen und pflanzlichen Zellen vor und können beispielsweise durch Extraktion gewonnen werden. In einer bevorzugten Ausführungsform werden im Rahmen dieser Erfindung nur Phospholipidgemische (ii) eingesetzt, die durch Extraktion aus pflanzlichen Bestandteilen, bevorzugt aus ausschließlich pflanzlichen Bestandteilen, gewonnen worden sind. Beispielsweise ist als extrahierbarer pflanzlicher Bestandteil Soja geeignet. Insbesondere wird ein Phospholipidgemisch (ii) verwendet, dass durch Extraktion von Soja erhalten wurde.

Das Phospholipidgemisch (ii), enthält die Bestandteile Phosphatidylcholin(Bestandteil ii-1), Phosphatidylethanolamin (Bestandteil ii-2) und gegebenenfalls Sphingomyelin (Bestandteil ii-3).

Phosphatidylcholin (im Rahmen dieser Erfindung auch als "PC" bezeichnet) ist im Fachgebiet bekannt. Es basiert auf einem Triglycerid, das zwei Fettsäurereste und einen Phosphorsäurerest, an den Cholin als Base gebunden ist, enthält. Phosphatidylcholin (ii-1) ist im Phospholipidgemisch (ii) in einer Menge von 60 bis 95 Gewichtsprozent (Gew.-%), bevorzugt von 65 bis 80 Gew.-%, besonders bevorzugt 68 bis 75 Gew.-%, bezogen auf das Gesamtgewicht des Phospholipidgemisches (ii), enthalten.

Phosphatidylethanolamin (im Rahmen dieser Erfindung auch als "PE" bezeichnet) ist im Fachgebiet bekannt. Es basiert auf einem Triglycerid, das zwei Fettsäurereste und einen Phosphorsäurerest, an den Colamin als Base gebunden ist, enthält. Phosphatidylethanolamin (ii-2) ist im Phospholipidgemisch (ii) in einer Menge von 2 bis 25 Gewichtsprozent (Gew.-%), bevorzugt von 5 bis 15 Gew.-%, besonders bevorzugt 6 bis 12 Gew.-%, bezogen auf das Gesamtgewicht des Phospholipidgemisches (ii), enthalten.

Sphingomyelin (im Rahmen dieser Erfindung auch als "Sph" bezeichnet) ist im Fachgebiet bekannt. Es ist analog zu Phosphatidylcholin aufgebaut, wobei jedoch das Glycerin durch Sphingosin (einem ungesättigten Aminoalkohol) ersetzt wurde, d.h. Sphingomyelin ist aufgebaut aus je einem Molekül Fettsäure, Sphingosin, Phosphorsäure und Cholin. Sphingomyelin (ii-3) ist ein optionaler Bestandteil im Phospholipidgemisch (ii). Sphingomyelin (ii-3) ist im allgemeinen im Phospholipidgemisch (ii) in einer Menge von 0 bis 15 Gewichtsprozent (Gew.-%), bevorzugt von 0,1 bis 5 Gew.-%, besonders bevorzugt 0,5 bis 3 Gew.-%, bezogen auf das Gesamtgewicht des Phospholipidgemisches (ii), enthalten.

Wie vorstehend beschrieben enthalten die Bestandteile (ii-1), (ii-2) und (ii-3) Fettsäuren in gebundener Form. Als Fettsäuren sind hier im allgemeinen Monocarbonsäuren mit 8 bis 25 Kohlenstoffatomen, bevorzugt von 12 bis 22 Kohlenstoffatomen, geeignet. Bevorzugt verwendet werden in den Bestandteilen (ii-1), (ii-2) und (ii-3) die Fettsäuren Palmitinsäure, Stearinsäure, Ölsäure, Linolsäure und Linolensäure.

In einer bevorzugten Ausführungsform weist die Fettsäurenzusammensetzung des Phospholipidgemisches (ii) einen molaren Anteil von
10 bis 30 %, insbesondere 15 bis 22 % Palmitinsäure,
0,1 bis 10 %, insbesondere 1 bis 7 % Stearinsäure,
3 bis 20 %, insbesondere 7 bis 15 % Ölsäure,
40 bis 80 % Linolsäure, mehr bevorzugt 45 bis 75, insbesondere 55 bis 70 %, und
1 bis 15 %, insbesondere 3 bis 8 % Linolensäure auf.

Neben den Bestandteilen (ii-1), (ii-2) und (ii-3) kann das Phospholipidgemisch weitere bekannte Phospholipide enthalten. Beispiele hierfür sind Phosphatidylserin, Phosphatidylglycerol und/oder Phospatidylinositol. Es ist jedoch bevorzugt, dass Phospholipide, die nicht unter die Bestandteile (ii-1), (ii-2) und (ii-3) fallen, nur in geringen Mengen, d.h. in einer Menge von weniger als 10 Gew.-%, mehr bevorzugt in einer Menge von weniger als 5 Gew.-%, insbesondere in einer Menge von weniger als 3 Gew.-%, bezogen auf das Gesamtgewicht des Phospholipidgemisches (ii), enthalten sind.

Neben den Bestandteilen (ii-1), (ii-2) und (ii-3) kann das Phospholipidgemisch weitere bekannte Lipidbestandteile enthalten. Beispiele hierfür sind Triglyceride, freie Fettsäuren und/oder α-Tocopherol. Es ist jedoch bevorzugt, dass Lipidbestandteile, die nicht unter die Bestandteile (ii-1), (ii-2) und (ii-3) fallen, nur in geringen Mengen, d.h. in einer Menge von weniger als 10 Gew.-%, mehr bevorzugt in einer Menge von weniger als 5 Gew.-%, insbesondere in einer Menge von weniger als 3 Gew.-%, bezogen auf das Gesamtgewicht des Phospholipidgemisches (ii), enthalten sind.

Des weiteren wird das Phospholipidgemisch (ii) so gewählt, dass es einen Phosphorgehalt von 3 bis 5 Gew.-%, bevorzugt von 3,1 bis 4,5 Gew.-%, besonders bevorzugt von 3,3 bis 3,8 %, bezogen auf das Gesamtgewicht des Phospholipidgemisches (ii), aufweist. Ferner das Phospholipidgemisch (ii) so gewählt, dass es eine Jodzahl von 70 bis 110, bevorzugt von 80 bis 100, insbesondere von 85 bis 95 aufweist.

Das Phospholipidgemisch (ii) wird im allgemeinen in einer Menge von 0,1 bis 12 Gew.-%, bevorzugt 0,2 bis 5 Gew.-%, besonders bevorzugt von 0,5 bis 3 Gew.-%, bezogen auf das Gesamtgewicht der erfindungsgemäßen Zusammensetzung, eingesetzt.

Die Zusammensetzung kann neben der wässrigen Lösung (i) und dem Phospholipidgemisch (ii) noch weitere Bestandteile enthalten. In einer bevorzugten Ausführungsform enthält die erfindungsgemäße Zusammensetzung neben der wässrigen Lösung (i) und dem Phospholipidgemisch (ii) zusätzlich eine oder mehrere Bestandteile, ausgewählt aus
(iii) einem mehrwertigen Alkohol,
(iv) einem natürlich vorkommenden Öl, und
(v) einem Alkalisalz einer Fettsäure.

Als Bestandteil (iii) kann ein mehrwertiger Alkohol verwendet werden, bevorzugt wird ein physiologisch verträglicher Alkohol verwendet, besonders bevorzugt wird Glycerin verwendet. Der mehrwertige Alkohol (iii) wird im allgemeinen in einer Menge von 0 bis 10 Gew.-%, bevorzugt 0,1 bis 5 Gew.-%, besonders bevorzugt von 0,2 bis 3 Gew.-%, bezogen auf das Gesamtgewicht der erfindungsgemäßen Zusammensetzung, eingesetzt.

Als Bestandteil (iv) kann ein natürlich vorkommendes Öl verwendet werden, bevorzugt wird ein pflanzliches Öl verwendet, beispielsweise Sojaöl, Sesamöl, Sonnenblumenöl oder Erdnussöl. Besonders bevorzugt wird Sojaöl verwendet. Das natürlich vorkommende Öl (iv) wird im allgemeinen in einer Menge von 0 bis 30 Gew.-%, bevorzugt 1 bis 20 Gew.-%, besonders bevorzugt von 2 bis 10 Gew.-%, bezogen auf das Gesamtgewicht der erfindungsgemäßen Zusammensetzung, eingesetzt.

Als Bestandteil (v) kann ein Alkalisalz einer Fettsäure verwendet werden, bevorzugt ein Natrium- oder Kaliumsalz einer Fettsäure mit 14 bis 18 Kohlenstoffatomen. Insbesondere wird Natriumoleat oder Kaliumoleat verwendet. Das Alkalisalz einer Fettsäure (v) wird im allgemeinen in einer Menge von 0 bis 5 Gew.-%, bevorzugt 0,1 bis 2 Gew.-%, besonders bevorzugt von 0,2 bis 1,5 Gew.-%, bezogen auf das Gesamtgewicht der erfindungsgemäßen Zusammensetzung, eingesetzt.

In einer bevorzugten Ausführungsform werden die Bestandteile (i) bis (v) der erfindungsgemäßen Zusammensetzung so gewählt, dass die erfindungsgemäße Zusammensetzung im wesentlichen nur körpereigene Substanzen oder solche Substanzen, die üblicherweise mit der Nahrung aufgenommen werden, enthält. Insbesondere werden die Bestandteile (i) bis (v) der erfindungsgemäßen Zusammensetzung so gewählt, dass die erfindungsgemäße Zusammensetzung keine Hyaluronsäure und kein Propylenglykol enthält.

Die erfindungsgemäße Zusammensetzung enthält die Bestandteile (i) und (ii) und gegebenenfalls (iii), (iv) und (v). Das Phospholipidgemisch (ii) liegt hierbei in Form von Teilchen, bevorzugt in Form Liposomen, in der wässrigen Lösung (i), die gegebenenfalls noch die Bestandteile (iii), (iv) und (v) enthält, vor. Der Begriff "Liposome" ist im Fachgebiet bekannt. Im allgemeinen werden darunter konzentrische Vesikel verstanden, die unilamellar oder multilamellar aufgebaut sein können.

In der vorliegenden Erfindung (ii) liegt das Phospholipidgemisch (ii) in Form von Teilchen, bevorzugt in Form von Liposomen, vor. Diese Teilchen können unterschiedliche Teilchendurchmesser aufweisen und liegen deshalb in Form einer Teilchengrößenverteilung vor.

In der vorliegenden Erfindung (ii) liegt das Phospholipidgemisch (ii) in Form von Teilchen, bevorzugt in Form von Liposomen, mit einem mittleren Teilchendurchmesser von 15 bis 250 nm, mehr bevorzugt 30 bis 150 nm, besonders bevorzugt 50 bis 130 nm vor.

Im Rahmen dieser Patentanmeldung wird der mittlere Teilchendurchmesser durch Photonenkorrelationsspektroskopie bestimmt. Die Photonenkorrelationsspektroskopie erfolgt mit einem Gerät Zeta Sizer 3000 der Firma Malvern Instruments Ltd.. Die Messung wurde gemäß Bedienungshandbuch, Version 1.0, Kapitel Größenmessung durchgeführt.

In einer bevorzugten Ausführungsform weisen die Teilchen, bevorzugt die Liposomen, des Phospholipidgemisches eine Oberflächenladung von -10 bis -60 Millivolt (mV), bevorzugt von -20 bis -40 mV auf.

Der Begriff "Oberflächenladung, wie er im Rahmen dieser Erfindung zu verstehen ist, wird in Müller, Rainer H.: "Zetapotential und Partikelladung in der Laborpraxis - Einführung in die Theorie, Praktische Messdurchführung, Dateninterpretation", 1996 beschrieben.

Im Rahmen dieser Patentanmeldung wird die Oberflächenladung wie beschrieben in Müller, Rainer H.: "Zetapotential und Partikelladung in der Laborpraxis - Einführung in die Theorie, Praktische Messdurchführung, Dateninterpretation", 1996; Praktische Messdurchführung, Seiten 117 ff. Kapitel 3 bestimmt.

Gegenstand der Erfindung ist ferner ein Verfahren zur Herstellung der erfindungsgemäßen Zusammensetzung, umfassend die Schritte:
(a) Einbringen des Phospholipidgemisches (ii) und gegebenenfalls der Bestandteile (iii), (iv) und/oder (v) in die wässrige Lösung (i) und gegebenenfalls anschließend
(b) Hochdruckhomogenisation oder Hochdruckextrusion der Zusammensetzung aus Schritt (a).

Im Schritt (a) werden das Phospholipidgemisch (ii) und gegebenenfalls die Bestandteile (iii), (iv) und/oder (v) in die wässrige Lösung (i) eingebracht und, beispielsweise durch Rühren, vermischt. Je nach Wahl der Bestandteile (i) bis (v) können Liposomen mit unterschiedlichen Teilchendurchmessern resultieren. Sofern der mittlere Teilchendurchmesser der Liposomen in der resultierenden Zusammensetzung nicht zwischen 15 und 150 nm liegt, schließt sich an den Schritt (a) des erfindungsgemäßen Verfahrens der Schritt (b) an, wobei eine Hochdruckhomogenisation oder Hochdruckextrusion der Zusammensetzung aus Schritt (a) erfolgt.

Schritt (b) kann jedoch auch erfolgen, sofern nach Schritt (a) bereits der mittlere Teilchendurchmesser zwischen 15 und 150 nm liegt.

Der Begriff "Hochdruckhomogenisation" ist im Fachgebiet bekannt. Im allgemeinen wird hier die zu behandelnde Zusammensetzung (aus Schritt (a)) ein oder mehrmals mit Druck, bevorzugt von 100 bis 1500 bar, besonders bevorzugt von 300 bis 1000 bar, durch Kapillare oder Ringspalte gepresst. Die bei diesem Prozess auftretenden Scherkräften zerkleinern im allgemeinen die in der Zusammensetzung enthaltenen Liposomen.

Der Begriff "Extrusion" ist ebenfalls im Fachgebiet bekannt. Im allgemeinen wird hier die zu behandelnde Zusammensetzung (aus Schritt (a)), beispielsweise mittels Schneckenförderung, durch eine Düse gepresst.

In einer bevorzugten Ausführungsform wird die Zusammensetzung während der Durchführung des Schrittes (b) gekühlt. Bevorzugt wird die Kühlung so geregelt, dass die Zusammensetzung während der Hochdruckhomogenisation oder während der Extrusion eine Temperatur von 10 bis 50 °C, bevorzugt von 15 bis 30 °C, aufweist.

Gegenstand der Erfindung ist ferner eine Zusammensetzung, enthaltend
(i) eine wässrige Lösung und
(ii) ein Phospholipidgemisch, enthaltend, bezogen auf das Gesamtgewicht des Phospholipidgemisches (ii),
(ii-1) 60 bis 95 Gewichtsprozent (Gew.-%) Phosphatidylcholin,
(ii-2) 2 bis 25 Gew.-% Phosphatidylethanolamin und
(iii-3) 0 bis 15 Gew.-% Sphingomyelin,
   wobei das Phospholipidgemisch in Form von Liposomen mit einem mittleren Teilchendurchmesser von 15 bis 150 nm in der wässrigen Lösung vorliegt, zur Verwendung als Arzneimittel.

Das erfindungsgemäße Arzneimittel wird bevorzugt parenteral als Injektionslösung verabreicht.

Die erfindungsgemäße Zusammensetzung kann zur Herstellung eines Arzneimittels zur Behandlung von Gelenkerkrankungen, insbesondere von Osteoarthrose, verwendet werden. Ferner kann die Zusammensetzung zur Herstellung eines Arzneimittels zur Verhinderung von unerwünschten Adhäsionen, insbesondere von postoperativen Adhäsionen, verwendet werden. Gegenstand der Erfindung ist somit die Verwendung der erfindungsgemäßen Zusammensetzung zur Herstellung eines Arzneimittels zur Behandlung von Gelenkerkrankungen, insbesondere von Osteoarthrose oder zur Verhinderung postoperativer Adhäsion.

Der Begriff "Osteoarthrose" ist im Fachgebiet bekannt. Es handelt sich hierbei um eine überwiegend chronische, degenerative Erkrankung der Gelenke.

Insbesondere wird im Rahmen dieser Erfindung unter dem Begriff "Osteoarthrose" eine Synthesestörung der aus Proteoglykanen und Kollagen zusammengesetzten organischen Matrix des Gelenkknorpels verstanden, d.h. beispielsweise eine geringere Permeabilität, Behinderung des Substratnachschubs, Verschlechterung der nutritiven Situation und/oder Absterben von Knorpelzellen. Weiterhin kann "Osteoarthrose" auch ein Nachlassen der Elastizität und Demaskierung des kollagenen Fasergerüsts umfassen. "Osteoarthrose" kann zugleich verstärkter Abbau durch Freisetzung und Einwirkung lysosomaler Enzyme bedeuten. Durch "Osteoarthrose" kann unter den degenerativen Knorpelveränderungen im gelenkbildenden Knochenabschnitt subchondrale Sklerosierung als Kompensationsmaßnahme im belasteten Gelenkanteil erfolgen, bei fortschreitendem Prozess Bildung von Geröllzysten oder anderen Formen der Knochenerosion sowie unterschiedlich starke Ausprägung von Osteophyten.

Der Begriff "postoperative Adhäsion" ist ebenfalls im Fachgebiet bekannt. Im allgemeinen wird darunter eine entzündlich bedingte, flächenhafte oder strangförmige, nach Verklebung durch Fibrin entstandene bindegewebige Verwachsung aneinander liegender, Serosa-überzogener Organabschnitte verstanden. Sie kann Verwachsungs- = "Adhäsionsbeschwerden" u. Funktionsstörungen (z.B. Adhäsionsileus; s.a. Bridenileus) bewirken. Dieses Ereignis kann z.B. durch eine Operation ausgelöst werden.

Die erfindungsgemäße Zusammensetzung kann zur Herstellung eines Arzneimittels zur Behandlung von Augenerkrankungen, insbesondere in Form von Augentropfen, verwendet werden. Gegenstand der Erfindung ist somit die Verwendung der erfindungsgemäßen Zusammensetzung zur Herstellung von Augentropfen.

Beispiele für Indikationen für Augentropfen:
Zur Linderung und/oder Besserung von trockenen Augen (auch bekannt unter dem Begriff "Dry eye syndrome").
Die erfindungsgemäße Zusammensetzung schützt und befeuchtet die Augenoberfläche und macht sie gleitfähig. Ferner kann die erfindungsgemäße Zusammensetzung als Augentropfen zur Linderung bei Trockenheitsgefühl und bei leichter, nicht als patholog. einzustufender Reizung durch umgebungsbedingte Befindlichkeitsstörungen, wie z. B. Brennen, Ermüdung, langes Arbeiten vor d. Bildschirm od. Tragen v. Kontaktlinsen, verwendet werden.

Die erfindungsgemäße Zusammensetzung kann zur Herstellung eines Arzneimittels zur Pertubation von nicht durchgängigen Tuben, beispielsweise von verklebten Eileitern, insbesondere in Form einer Tubenspülung, verwendet werden. Gegenstand der Erfindung ist somit die Verwendung der erfindungsgemäßen Zusammensetzung zur Herstellung einer Tubenspülung. Insbesondere wird die erfindungsgemäße Zusammensetzung als Tubenspülung in Eileitern verwendet, die durch Pertubation durchgängig gemacht wurden, um ein erneutes Verkleben der Eileiter zu verhindern.

Die vorliegende Erfindung soll anhand nachstehender Beispiele veranschaulicht werden.

### Beispiele:

In Tabelle 2 sind die Beispiele 1 bis 9 für erfindungsgemäße Zusammensetzungen dargestellt. Die Zusammensetzung der in Tabelle 2 verwendeten Phospholipidgemische A und B ist in Tabelle 1 dargestellt.

Die Herstellung der erfindungsgemäßen Zusammensetzung erfolgte durch folgendes Verfahren:

### 1. Betriebsmittel

Es wurde ein Ansatzbehälter mit Rührwerk, Temperiereinheit, Hochdruckhomogenisator LAB-60 bzw. LAB 40 (für kleinere Ansätze) (Firma APV Deutschland GmbH, 59425 Unna) und ein Filter mit Filtergehäuse verwendet.

### 2. Durchführung

*Schritt a-1:*
Im Ansatzkessel wurde Wasser für Injektionszwecke vorgelegt.
Das Rührwerk wurde eingeschaltet.
Nacheinander wurden gegebenenfalls die Puffersubstanzen und Bestandteile (iii) bis (v) gemäß Tabelle 2 zugegeben und bis zur vollständigen Lösung gerührt.
*Schritt a-2:*
Der pH-Wert der Lösung wurde überprüft und gegebenenfalls durch Zugabe von Säure oder Base angepasst. Für die in Tabelle 2 dargestellten Zusammensetzungen wurde ein pH-Wert von 7,4 eingestellt.
*Schritt a-3:*
Die Phospholipidgemisch A beziehungsweise B gemäß Tabelle 2 wurden zugegeben und es wurde bis zur Lösung des Phospholipidgemisches gerührt. Es wurde eine trübe gelbliche Lösung erhalten, wobei optisch keine Phospholipidpartikel erkennbar waren.
*Schritt b-1:*
Es erfolgte eine Homogenisation der Zusammensetzung mittels Hochdruckhomogenisator. Es wurden 3 Zyklen ä 500 bar gefahren. Die Lösung wurde während der Homogenisation gekühlt.
*Schritt b-2:*
Es erfolgte Filtration der Zusammensetzung und anschließende Abfüllung in Ampullen. Man erhielt eine leicht trübe, gelbliche Lösung, ohne optisch erkennbare Partikel. Keine Bildung von Bodensatz wurde beobachtet.
Zur Filtration wurde folgender Filter verwendet:

| | |
|---|---|
| Firma: | Millipore |
| Typ: | Optiseal Durapore |
| Porengröße: | 0,22µm |
| Material: | Polypropylen |

**Tabelle 1:**

| | **A** | **B** |
|---|---|---|
| Phosphatidylcholin | 73 % | 80 % |
| Phosphatidyethanolamin | 7-10% | 10 % |
| Sphingomyelin | 0 % | 3 % |

Tabelle 1 zeigt die Zusammensetzung der in Tabelle 2 verwendeten Phospholipidgemische A und B (Angaben in Gewichtsprozent, bezogen auf das Gesamtgewicht der Phospholipidgemisches A und B). Das Phospholipidgemisch A wurde durch Extraktion aus Soja gewonnen, das Phospholipidgemisch B wurde durch Extraktion aus Ei gewonnen.

**Tabelle 2: Erfindungsgemäße Beispiele 1 bis 9**

| **Bsp.** | **Lipid A** | **Lipid B** | **H**_{**3**}**PO**_{**4**} | **Glycerin** | **Soja-öl** | **Na-Oleat** | **Tris** | **Na**_{**2**}**HPO**_{**4**}**/ NaH**_{**2**}**PO**_{**4**} | **H**_{**2**}**O** |
|---|---|---|---|---|---|---|---|---|---|
| **1** | 0 | 1,2 | - | 2,5 | 10 | 0,05 | - | - | ad 100 |
| **2** | 0 | 0,5 | q.s. | 0 | 0 | 0 | - | 0,26 | ad 100 |
| **3** | 0,1 | 0 | q.s. | 0 | 0 | 0 | - | 0,26 | ad 100 |
| **4** | 0,5 | 0 | q.s. | 0 | 0 | 0 | - | 0,26 | ad 100 |
| **5** | 0 | 0,5 | q.s. | 2,5 | 0 | 0 | - | 0,26 | ad 100 |
| **6** | 2,5 | 0 | q.s. | 0 | 0 | 0 | - | 0,26 | ad 100 |
| **7** | 5 | 0 | q.s. | 0 | 0 | 0 | - | 0,26 | ad 100 |
| **8** | 0,5 | 0 | q.s. | 2,4 | 0 | 0 | 0,02 | 0 | ad 100 |
| **9** | 0,5 | 0 | q.s. | 2,4 | 0 | 0 | 0 | 0,046* | ad 100 |

| | | | | | | | | | |
|---|---|---|---|---|---|---|---|---|---|
| *= K₂HPO₄/KH₂PO₄ | | | | | | | | | |

In Tabelle 2 bedeutet "q.s." eine ausreichende Menge, um den gewünschten pH-Wert von 7,4 zu erreichen.

### Reibungsversuche:

Die Beispiele 1, 2 und 4 bis 6 wurden Reibungsversuchen unterzogen. Hierbei wurde Wasser und trockenen Platten (d.h. kein Mittel zur Verminderung der Reibung) als Referenz angeführt.

Getestet wurde die Reibungsverminderung über einen Geschwindigkeitsgradienten. Die Belastung war 6 Newton. Die Materialpaarung war PE-PE (PE= Polyethylen). Die Ergebnisse sind in Diagramm 3 dargestellt.

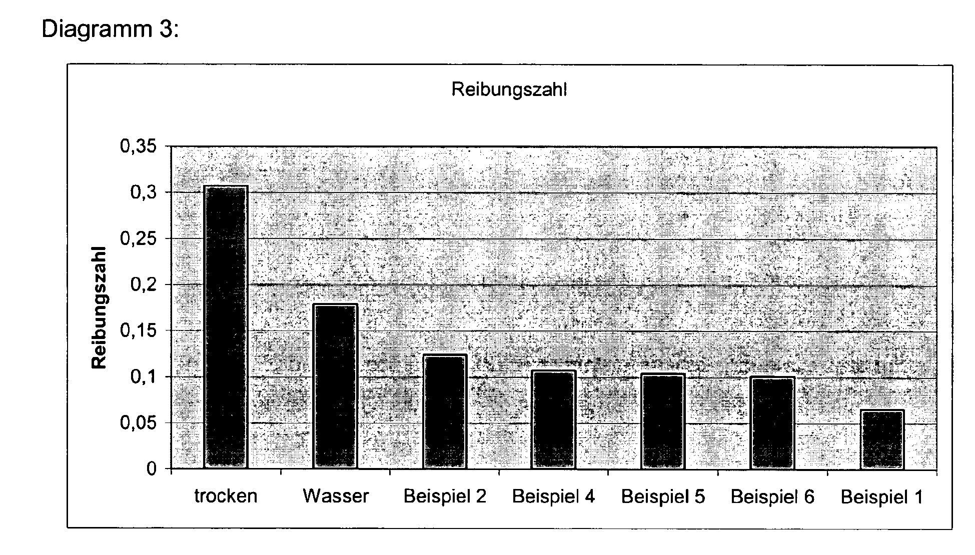

## Patentansprüche

1. Zusammensetzung, enthaltend
(i) eine wässrige Lösung und
(ii) ein Phospholipidgemisch, enthaltend, bezogen auf das Gesamtgewicht des Phospholipidgemisches (ii),
(ii-1) 60 bis 95 Gewichtsprozent (Gew.-%) Phosphatidylcholin,
(ii-2) 2 bis 25 Gew.-% Phosphatidylethanolamin und
(iii-3) 0 bis 15 Gew.-% Sphingomyelin,
wobei das Phospholipidgemisch (ii) in Form von Teilchen mit einem mittleren Teilchendurchmesser von 15 bis 250 nm, bevorzugt von 30 bis 150 nm, besonders bevorzugt von 50 bis 130 nm, in der wässrigen Lösung (i) vorliegt.

2. Zusammensetzung nach Anspruch 1, **dadurch gekennzeichnet, dass** die Teilchen eine Oberflächenladung von -10 bis -60 mV aufweisen.

3. Zusammensetzung nach Anspruch 1 oder 2, **dadurch gekennzeichnet, dass** die wässrige Lösung (i) in einem pH-Bereich von 6 bis 9 gepuffert ist.

4. Zusammensetzung nach einem der Ansprüche 1 bis 3, **dadurch gekennzeichnet, dass** das Phospholipidgemisch (ii) durch Extraktion von pflanzlichen Bestandteilen erhalten wurde.

5. Zusammensetzung einem der Ansprüche 1 bis 4, **dadurch gekennzeichnet, dass** das Phospholipidgemisch (ii) in einer Menge von 0,1 bis 12 Gew.-%, bevorzugt 0,2 bis 5 Gew.-%, bezogen auf das Gesamtgewicht der Zusammensetzung, vorliegt.

6. Zusammensetzung nach einem der Ansprüche 1 bis 5, **dadurch gekennzeichnet, dass** diese zusätzlich eine oder mehrere Bestandteile, ausgewählt aus
(iii) einem mehrwertigen Alkohol, bevorzugt Glycerin,
(iv) einem natürlich vorkommenden Öl, bevorzugt Sojaöl, und
(v) einem Alkalisalz einer Fettsäure, bevorzugt Natriumoleat, enthält.

7. Zusammensetzung nach einem der Ansprüche 1 bis 6, **dadurch gekennzeichnet, dass** der mehrwertige Alkohol (iii) in einer Menge von 0 bis 5 Gew.-%, bevorzugt 0,1 bis 3 Gew.-%, bezogen auf das Gesamtgewicht der Zusammensetzung, vorliegt.

8. Zusammensetzung nach einem der Ansprüche 1 bis 7, **dadurch gekennzeichnet, dass** das natürlich vorkommende Öl (iv) in einer Menge von 0 bis 30 Gew.-%, bevorzugt von 1 bis 20 Gew.-%, bezogen auf das Gesamtgewicht der Zusammensetzung, vorliegt.

9. Zusammensetzung nach einem der Ansprüche 1 bis 8, **dadurch gekennzeichnet, dass** das Alkalisalz einer Fettsäure (v) in einer Menge von 0 bis 5 Gew.-%, bevorzugt von 0,1 bis 2 Gew.-%, bezogen auf das Gesamtgewicht der Zusammensetzung, vorliegt.

10. Zusammensetzung nach einem der Ansprüche 1 bis 9, **dadurch gekennzeichnet, dass** die Fettsäurenzusammensetzung des Phospholipidgemisches (ii) einen molaren Anteil von
10 bis 30 % Palmitinsäure,
0,1 bis 10 % Stearinsäure,
3 bis 20 % Ölsäure,
40 bis 80 % Linolsäure und
1 bis 15 % Linolensäure,
aufweist.

11. Zusammensetzung nach einem der Ansprüche 1 bis 10 zur Verwendung als Arzneimittel.

12. Verfahren zur Herstellung einer Zusammensetzung gemäß einem der Ansprüche 1 bis 11, umfassend die Schritte:
(a) Einbringen des Phospholipidgemisches (ii) und gegebenenfalls der Bestandteile (iii), (iv) und/oder (v) in die wässrige Lösung (i) und gegebenenfalls anschließend
(b) Hochdruckhomogenisation oder Hochdruckextrusion der Zusammensetzung aus Schritt (a).

13. Verwendung einer Zusammensetzung gemäß einem der Ansprüche 1 bis 10 zur Herstellung eines Arzneimittels zur Behandlung von Osteoarthrose oder zur Verhinderung postoperativer Adhäsion.

14. Verwendung einer Zusammensetzung gemäß einem der Ansprüche 1 bis 10 zur Herstellung von Augentropfen.

15. Verwendung einer Zusammensetzung gemäß einem der Ansprüche 1 bis 10 zur Herstellung einer Tubenspülung.
